# EUROPEAN PATENT APPLICATION

(11) **EP 4 140 494 A1**
(43) Date of publication of application: **01.03.2023**
(21) Application number: 21792617.9
(22) Date of filing: 29.01.2021
(51) Int. Cl.: A61K 35/747, A61P 3/06, A61P 3/04, A61P 1/16, A23L 33/135

(54) **COMPOSITION FOR PREVENTING OR TREATING LIPID-RELATED METABOLIC DISEASES, COMPRISING LACTOBACILLUS PLANTARUM ATG-K2 OR ATG-K6**

(30) Priority: 21.04.2020 KR 20200048174
(71) Applicant: Atogen Co., Ltd., Daejeon 34015 (KR)
(72) Inventor: LEE, Hae-Jeung, Seongnam-si Gyeonggi-do 13556 (KR); PARK, Eun-Jung, Hanam-si Gyeonggi-do 13012 (KR); KANG, Ji-Hee, Daejeon 34010 (KR); BAEK, Hyun-Guh, Sejong-si 30064 (KR); PARK, Gun-Seok, Daejeon 34010 (KR)
(74) Representative: Gulde & Partner
(86) International application number: PCT/KR2021/001255
(87) International publication number: WO 2021/215627

(57) **Abstract**

Provided is a composition for preventing and treating lipid-related metabolic diseases, the composition comprising, as an active ingredient, Lactobacillus plantarum ATG-K2 or Lactobacillus plantarum ATG-K6 isolated from fermented vegetables.

## Description

### [Technical Field]

The present disclosure relates to a composition for preventing and treating lipid-related metabolic diseases, comprising, as an active ingredient, Lactobacillus plantarum ATG-K2 or ATG-K6, and more particularly, to a composition capable of being used for prevention and treatment of lipid-related metabolic diseases by containing Lactobacillus plantarum ATG-K2 or ATG-K6 as an active ingredient.

### [Background Art]

An increase in metabolic diseases such as chronic liver disease, Type 2 diabetes and heart disease is associated with an increased obesity rate. About 90% of patients with severe obesity are diagnosed with non-alcoholic fatty liver disease (NAFLD), 37% of the patients are diagnosed with non-alcoholic steatohepatitis, and 10% of the patients are diagnosed with cirrhosis.

Non-alcoholic fatty liver disease (NAFLD) is characterized by the accumulation of fat in 5% or more of adipocytes without excessive alcohol intake, which is the most common liver disease. Although this condition usually causes no symptoms in its early stages, the condition may be developed to serious liver disease, including liver fibrosis, cirrhosis, and cancer.

Sugars, especially fructose, may activate a hepatic lipogenesis program, which exacerbates the NAFLD. The fructose is processed almost exclusively in the liver and is mainly metabolized to triglycerides by de novo lipogenesis (DNL) in the liver. A fructose-derived precursor acts as a nutritional regulator of a transcription factor such as SREBP-1c and C/EBPα, and regulates of the expression of gluconeogenesis and a DNL gene. Fructose intake increases hepatic gluconeogenesis and DNL, and increases blood glucose and triglyceride levels.

Another cause of the non-alcoholic fatty liver disease (NAFLD) is oxidative stress. The accumulation of fat in the liver causes lipid peroxidation and promotes various responses, such as inflammation and fibrosis. Then, in the related art, antioxidants such as silymarin or vitamin E have been suggested as therapeutic agents for the non-alcoholic fatty liver disease (NAFLD). However, no standard pharmacological drug for the non-alcoholic fatty liver disease (NAFLD) has yet been developed.

Meanwhile, as known so far, probiotics mainly function to inhibit harmful bacteria in the intestine and help smooth bowel movements, and have been reported to help in skin moisturizing, maintaining skin health from skin damage caused by UV rays, female vaginal health, improvement of skin conditions due to immune hypersensitivity, and reduction of body fat, and has also been found to have an effect on psychiatric disorders.

Currently, the probiotics are focused on immunity and intestinal health research, but are related to improvement of antioxidant, anti-inflammatory, and immunity to have an effect of improving a liver function, but there is a lack of research data thereon.

Lactobacillus plantarum, which plays an important role in the production of various fermented foods, is one of the most important species of lactic acid bacteria (LAB). Lactobacillus plantarum is found in various environments, such as soil and human intestine, and has been considered to have the potential of probiotics.

Accordingly, the present inventors have conducted various studies on a method for improving lipid-related metabolic diseases using lactic acid bacteria, confirmed an excellent effect of prevention and treatment of lipid-related metabolic diseases by Lactobacillus plantarum ATG-K2 or ATG-K6, and then completed the present disclosure.

### [Disclosure]

### [Technical Problem]

An object of the present disclosure is to provide a composition for preventing and treating lipid-related metabolic diseases, including, as an active ingredient, Lactobacillus plantarum ATG-K2 or ATG-K6 isolated from fermented vegetables.

Another object of the present disclosure is to provide a health food composition for preventing and improving lipid-related metabolic diseases, including, as an active ingredient, Lactobacillus plantarum ATG-K2 or ATG-K6 isolated from fermented vegetables.

### [Technical Solution]

An aspect of the present disclosure provides a composition for preventing and treating lipid-related metabolic diseases containing, as an active ingredient, at least one selected from the group consisting of a strain of Lactobacillus plantarum ATG-K2 (accession No. KCTC 13577BP), a culture product of the strain, and a concentrate and a dry matter of the culture product.

Another aspect of the present disclosure provides a composition for preventing and treating lipid-related metabolic diseases containing, as an active ingredient, at least one selected from the group consisting of a strain of Lactobacillus plantarum ATG-K6 (accession No. KCTC 13570BP), a culture product of the strain, and a concentrate and a dry matter of the culture product.

Yet another aspect of the present disclosure provides a health food composition for preventing and improving lipid-related metabolic diseases containing, as an active ingredient, at least one selected from the group consisting of a strain of Lactobacillus plantarum ATG-K2 (accession No. KCTC 13577BP), a culture product of the strain, and a concentrate and a dry matter of the culture product.

Still another aspect of the present disclosure provides a health food composition for preventing and improving lipid-related metabolic diseases containing, as an active ingredient, at least one selected from the group consisting of a strain of Lactobacillus plantarum ATG-K6 (accession No. KCTC 13570BP), a culture product of the strain, and a concentrate and a dry matter of the culture product.

The composition may reduce a body weight gain rate and body fat, which are obesity indexes.

The composition may reduce enzyme activities of ALT, AST and ALP, which are liver damage indexes.

The composition may decrease serum triglyceride, serum total cholesterol, fasting serum glucose and leptin, and increase blood HDL cholesterol, adiponectin, superoxide dismutase (SOD), glutathione peroxidase (GPx) and catalse (CAT) .

The composition may decrease hepatic triglyceride and hepatic total cholesterol, and decrease hepatic lipid peroxidation (MDA).

The composition may decrease the expression of sterol regulatory element binding transcription factor 1 (SREBP-1c) mRNA, CCAAT/enhancer-binding protein alpha (C/EBP-α) mRNA and fatty acid synthase (FAS) mRNA, which are lipogenesis-related indexes.

The composition may decrease the expression of acetyl coA carboxylase (ACC) mRNA, which is a fatty acid oxidation-related index, and increase the expression of Carnitine palmitoyltransferase 1 (CPT-1) mRNA.

The composition may increase a Bacteroidetes group and inhibit a Firmicutes group in an intestinal flora.

### [Advantageous Effects]

According to the present disclosure, the present disclosure relates to a composition for preventing and treating lipid-related metabolic diseases, including, as an active ingredient, Lactobacillus plantarum ATG-K2 or ATG-K6 isolated from fermented vegetables. As a result of measuring a liver health-related index and an anti-obesity index by oral administration of Lactobacillus plantarum ATG-K2 or ATG-K6, it can be easily used as a composition for preventing and treating lipid-related metabolic diseases because of its excellent effect of improving the indexes.

According to the present disclosure, the present disclosure relates to a health food composition for preventing and improving lipid-related metabolic diseases, including, as an active ingredient, Lactobacillus plantarum ATG-K2 or ATG-K6 isolated from fermented vegetables. As a result of measuring a liver health-related index and an anti-obesity index by oral administration of Lactobacillus plantarum ATG-K2 or ATG-K6, it can be easily used as a health food composition for preventing and improving lipid-related metabolic diseases because of its excellent effect of improving the indexes.

### [Description of Drawings]

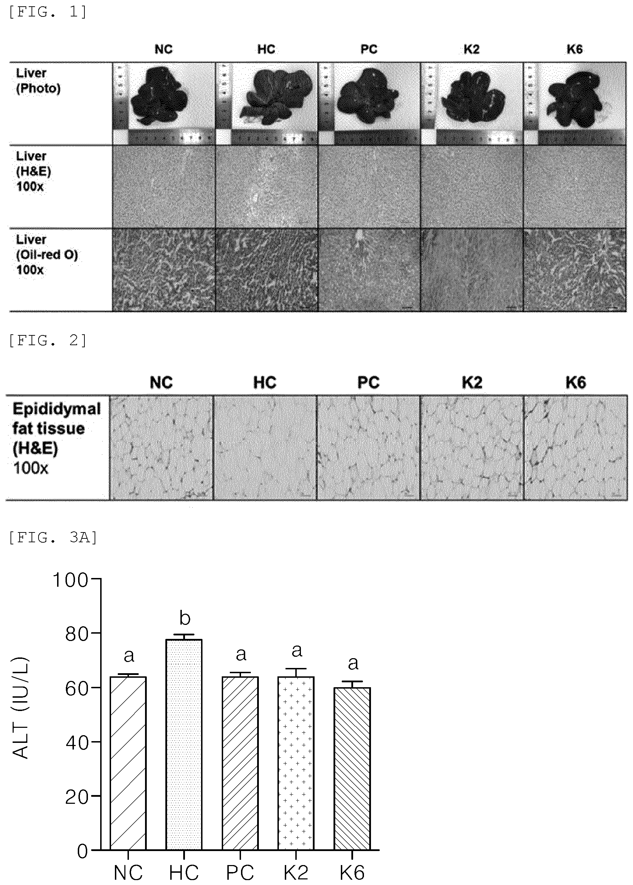

FIG. 1 is a photograph of liver tissue of high fat/high fructose dieted rats administered with Lactobacillus plantarum ATG-K2 or ATG-K6 and controls (NC, normal diet control; HC, HF/HF diet control; PC, HF/HF diet with silymarin; K2, HF/HF diet with ATG-K2; K6, HF/HF diet with ATG-K6, the same as below).
FIG. 2 is a photograph of epididymal fat of high fat/high fructose dieted rats administered with Lactobacillus plantarum ATG-K2 or ATG-K6 and controls.

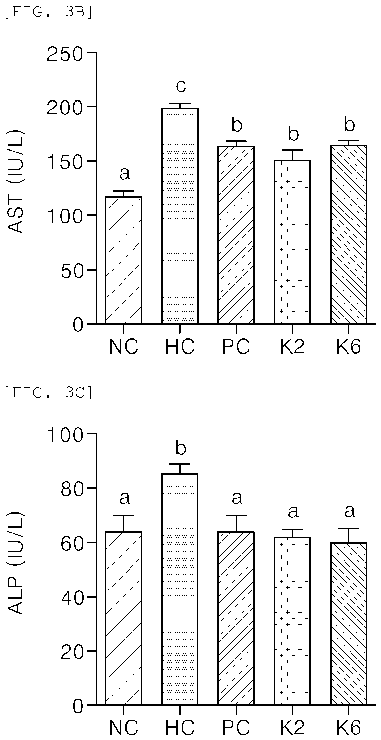

FIG. 3A is a graph showing a result of measuring ALT enzyme activity of high fat/high fructose dieted rats administered with Lactobacillus plantarum ATG-K2 or ATG-K6 and controls, FIG. 3B is a graph showing a result of measuring AST enzyme activity of high fat/high fructose dieted rats administered with Lactobacillus plantarum ATG-K2 or ATG-K6 and controls, and FIG. 3C is a graph showing a result of measuring ALP enzyme activity of high fat/high fructose dieted rats administered with Lactobacillus plantarum ATG-K2 or ATG-K6 and controls.

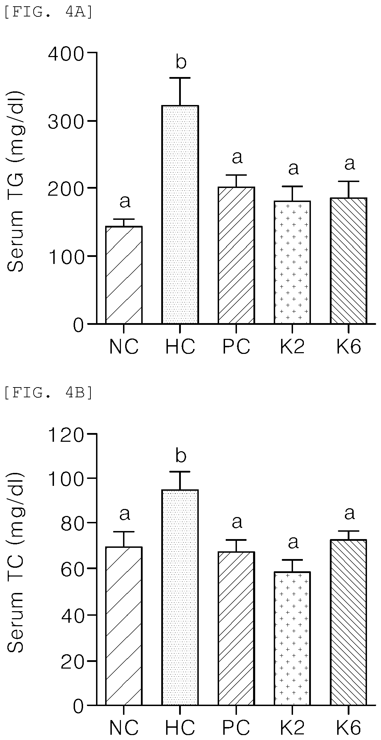

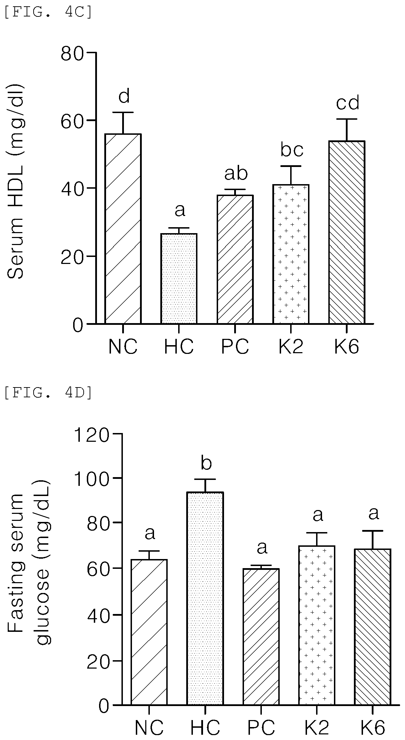

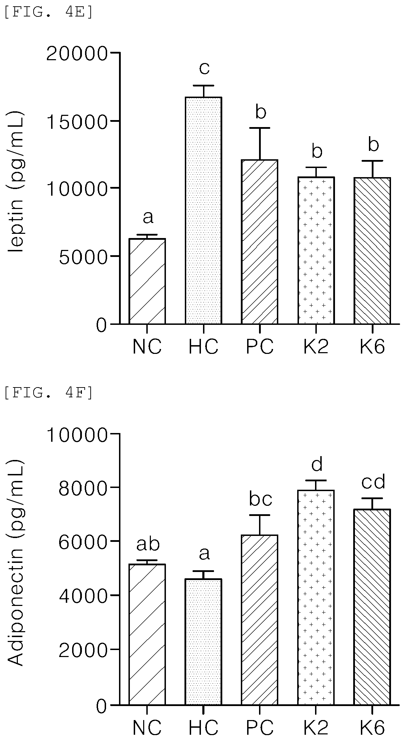

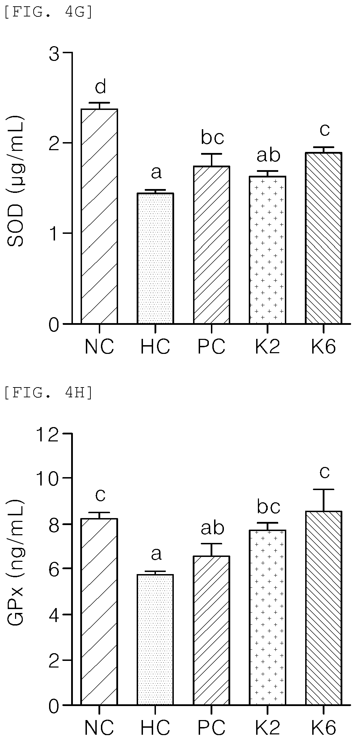

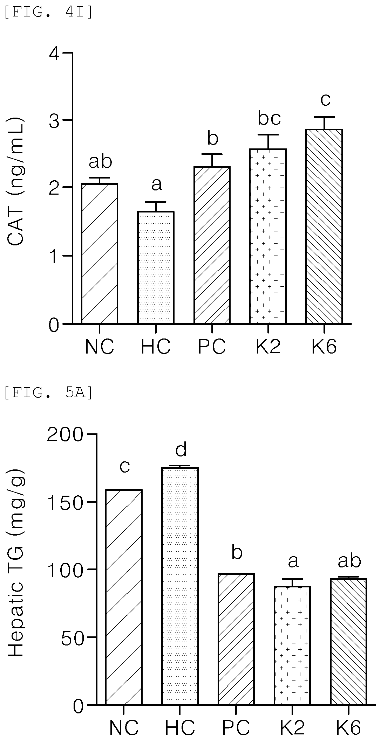

FIG. 4A is a graph showing a result of measuring serum triglyceride of high fat/high fructose dieted rats administered with Lactobacillus plantarum ATG-K2 or ATG-K6 and controls, FIG. 4B is a graph showing a result of measuring serum total cholesterol of high fat/high fructose dieted rats administered with Lactobacillus plantarum ATG-K2 or ATG-K6 and controls, FIG. 4C is a graph showing a result of measuring serum HDL cholesterol of high fat/high fructose dieted rats administered with Lactobacillus plantarum ATG-K2 or ATG-K6 and controls, FIG. 4D is a graph showing a result of measuring fasting serum glucose of high fat/high fructose dieted rats administered with Lactobacillus plantarum ATG-K2 or ATG-K6 and controls, FIG. 4E is a graph showing a result of measuring leptin of high fat/high fructose dieted rats administered with Lactobacillus plantarum ATG-K2 or ATG-K6 and controls, FIG. 4F is a graph showing a result of measuring adiponectin of high fat/high fructose dieted rats administered with Lactobacillus plantarum ATG-K2 or ATG-K6 and controls, FIG. 4G is a graph showing a result of measuring SOD of high fat/high fructose dieted rats administered with Lactobacillus plantarum ATG-K2 or ATG-K6 and controls, FIG. 4H is a graph showing a result of measuring GPx of high fat/high fructose dieted rats administered with Lactobacillus plantarum ATG-K2 or ATG-K6 and controls, and FIG. 4I is a graph showing a result of measuring CAT of high fat/high fructose dieted rats administered with Lactobacillus plantarum ATG-K2 or ATG-K6 and controls.

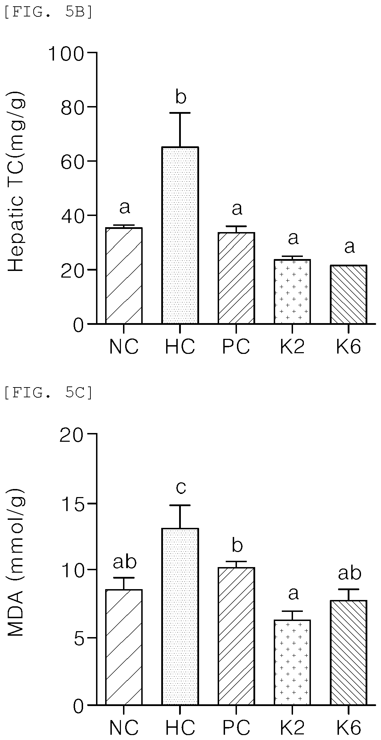

FIG. 5A is a graph showing a result of measuring hepatic triglyceride of high fat/high fructose dieted rats administered with Lactobacillus plantarum ATG-K2 or ATG-K6 and controls, FIG. 5B is a graph showing a result of measuring hepatic total cholesterol of high fat/high fructose dieted rats administered with Lactobacillus plantarum ATG-K2 or ATG-K6 and controls, and FIG. 5C is a graph showing a result of measuring hepatic lipid peroxidation (MDA) of high fat/high fructose dieted rats administered with Lactobacillus plantarum ATG-K2 or ATG-K6 and controls.

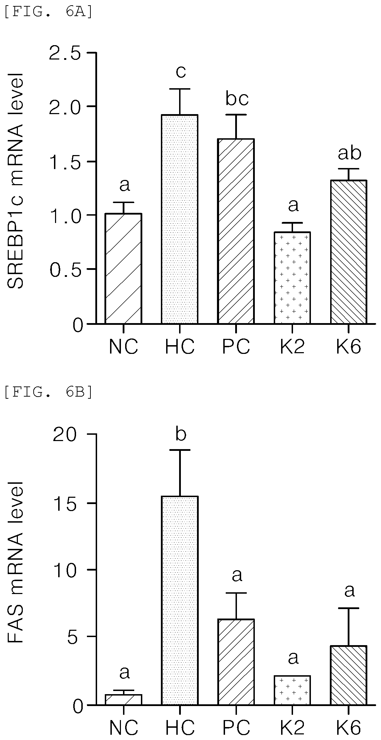

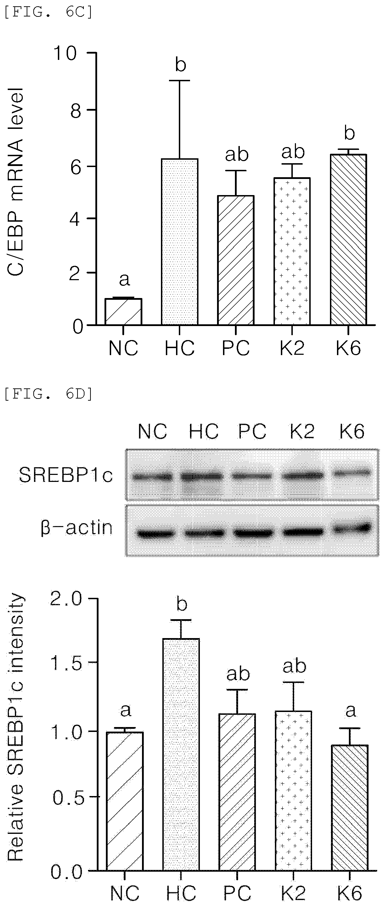

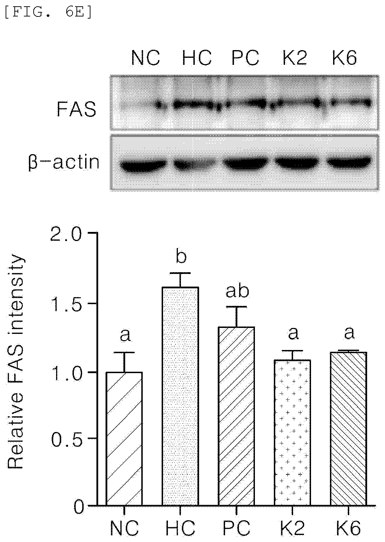

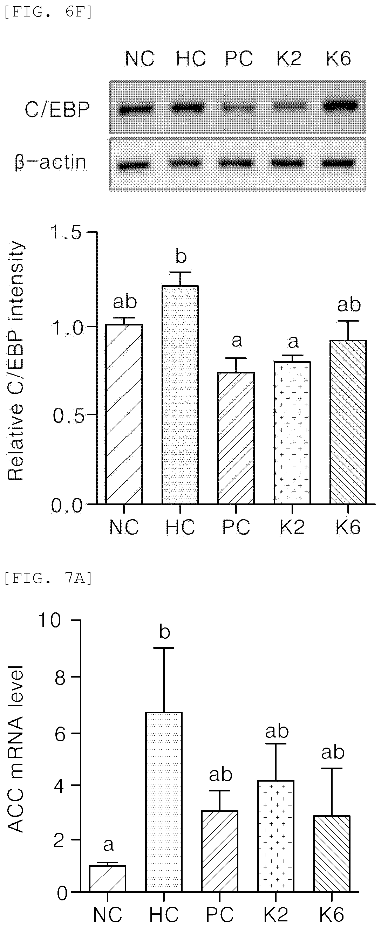

FIG. 6A illustrates mRNA expression levels of SREBP1c in high fat/high fructose dieted rats administered with Lactobacillus plantarum ATG-K2 or ATG-K6 and controls, FIG. 6B illustrates mRNA expression levels of FAS in high fat/high fructose dieted rats administered with Lactobacillus plantarum ATG-K2 or ATG-K6 and controls, FIG. 6C illustrates mRNA expression levels of C/EBP in high fat/high fructose dieted rats administered with Lactobacillus plantarum ATG-K2 or ATG-K6 and controls, FIG. 6D illustrates Western blot analysis results of SREBP1c in high fat/high fructose dieted rats administered with Lactobacillus plantarum ATG-K2 or ATG-K6 and controls, FIG. 6E illustrates Western blot analysis results of FAS in high fat/high fructose dieted rats administered with Lactobacillus plantarum ATG-K2 or ATG-K6 and controls, and FIG. 6F illustrates Western blot analysis results of C/EBP in high fat/high fructose dieted rats administered with Lactobacillus plantarum ATG-K2 or ATG-K6 and controls.

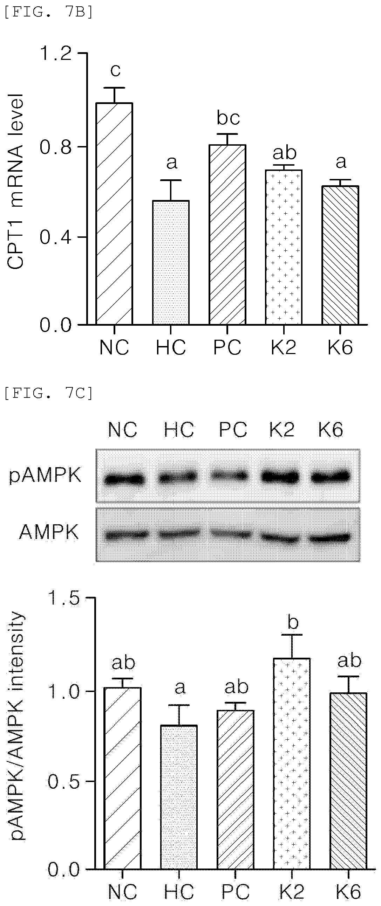

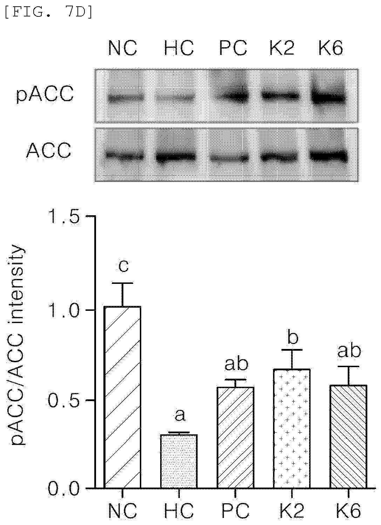

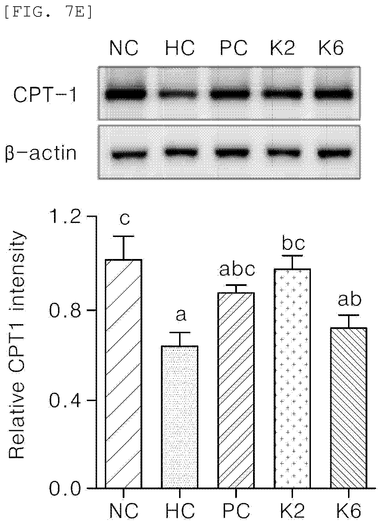

FIG. 7A illustrates mRNA expression levels of ACC in high fat/high fructose dieted rats administered with Lactobacillus plantarum ATG-K2 or ATG-K6 and controls, FIG. 7B illustrates mRNA expression levels of CPT-1 in high fat/high fructose dieted rats administered with Lactobacillus plantarum ATG-K2 or ATG-K6 and controls, FIG. 7C illustrates Western blot analysis results of phosphorylated AMPK and total AMPK in the liver in high fat/high fructose dieted rats administered with Lactobacillus plantarum ATG-K2 or ATG-K6 and controls, FIG. 7D illustrates Western blot analysis results of phosphorylated ACC and total ACC in the liver in high fat/high fructose dieted rats administered with Lactobacillus plantarum ATG-K2 or ATG-K6 and controls, and FIG. 7E illustrates Western blot analysis results of CPT-1 in the liver in high fat/high fructose dieted rats administered with Lactobacillus plantarum ATG-K2 or ATG-K6 and controls.

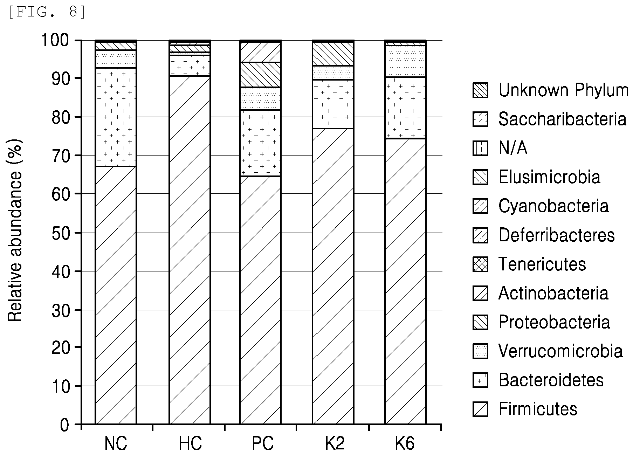

FIG. 8 is a graph showing differences in intestinal flora in high fat/high fructose dieted rats administered with Lactobacillus plantarum ATG-K2 or ATG-K6 and controls.

### [Best Mode]

The present disclosure provides a composition for preventing and treating lipid-related metabolic diseases containing, as an active ingredient, at least one selected from the group consisting of a strain of Lactobacillus plantarum ATG-K2 (accession No. KCTC 13577BP), a culture product of the strain, and a concentrate and a dry matter of the culture product.

Further, the present disclosure provides a composition for preventing and treating lipid-related metabolic diseases containing, as an active ingredient, at least one selected from the group consisting of a strain of Lactobacillus plantarum ATG-K6 (accession No. KCTC 13570BP), a culture product of the strain, and a concentrate and a dry matter of the culture product.

Further, the present disclosure provides a health food composition for preventing and improving lipid-related metabolic diseases containing, as an active ingredient, at least one selected from the group consisting of a strain of Lactobacillus plantarum ATG-K2, a culture product of the strain, and a concentrate and a dry matter of the culture product.

Further, the present disclosure provides a health food composition for preventing and improving lipid-related metabolic diseases containing, as an active ingredient, at least one selected from the group consisting of a strain of Lactobacillus plantarum ATG-K6, a culture product of the strain, and a concentrate and a dry matter of the culture product.

### [Mode for Invention]

In the following description, only parts required to understand exemplary embodiments of the present disclosure will be described, and it should be noted that the description of other parts will be omitted within a range without departing from the gist of the present disclosure.

Terms and words used in the present specification and claims should not be interpreted as being limited to typical or dictionary meanings, but should be interpreted as meanings and concepts which comply with the technical spirit of the present disclosure, based on the principle that the present inventor can appropriately define the concepts of the terms to describe his/her own invention in the best manner. Therefore, the exemplary embodiments described in the present specification and the configurations illustrated in the drawings are merely the most preferred exemplary embodiment of the present disclosure and are not intended to represent all of the technical ideas of the present disclosure, and thus, it should be understood that various equivalents and modifications capable of replacing the exemplary embodiments at the time of this application.

Hereinafter, the present disclosure will be described in detail.

The present disclosure provides a composition for preventing and treating lipid-related metabolic diseases containing, as an active ingredient, at least one selected from the group consisting of a strain of Lactobacillus plantarum ATG-K2, a culture product of the strain, and a concentrate and a dry matter of the culture product.

The present disclosure provides a composition for preventing and treating lipid-related metabolic diseases containing, as an active ingredient, at least one selected from the group consisting of a strain of Lactobacillus plantarum ATG-K6, a culture product of the strain, and a concentrate and a dry matter of the culture product.

Lactic acid bacteria including Lactobacillus plantarum ATG-K2 or ATG-K6 according to the present disclosure belong to GRAS (Generally Recognized As Safe), and the stability thereof has already been proven over a long period of time.

The Lactobacillus plantarum ATG-K2 or ATG-K6 according to the present disclosure belongs to probiotics announced by the Ministry of Food and Drug Safety as functionality 'capable of helping smooth bowel movement of proliferating lactic acid bacteria and suppressing harmful bacteria' .

The Lactobacillus plantarum ATG-K2 or ATG-K6 according to the present disclosure is lactic acid bacteria derived from fermented vegetables in the Chungcheong region.

In the present disclosure, the term 'culture product of the strain' is meant to include a culture medium, for example, a culture solution itself cultured in a liquid medium, a supernatant (filtrate) obtained by filtering and/or centrifuging the culture solution to remove the strain, and the like.

In the present disclosure, the 'concentrate and dry matter of the culture product' may be subjected to centrifugation or filtration to remove the liquid culture medium from the culture product and recover only the concentrated cells, but the present disclosure is not limited thereto. In addition, the concentrated cells may be dried, frozen, or freeze-dried according to a conventional method to be stored so as not to lose their activity.

Korean Patent Registration No. 10-1930438 discloses that Lactobacillus plantarum ATG-K2, ATG-K6 or ATG-K8 is effective in preventing and improving vaginitis in women, but the present disclosure has confirmed that Lactobacillus plantarum ATG-K2 or ATG-K6 is effective in preventing, improving and treating lipid-related metabolic diseases, and then completed the present disclosure.

The lipid-related metabolic diseases to be prevented or treated by the composition may be diabetes, hyperlipidemia, fatty liver, arteriosclerosis, hypertension, or cardiovascular disease, but is not limited thereto. It is known that the most fundamental cause is obesity due to the living environment, excessive nutrition intake, and insufficient energy consumption. Obesity refers to a state in which adipocytes proliferate and differentiate in vivo, resulting in excessive accumulation of fat, and means that when the absorbed amount of energy is relatively increased compared to the consumed amount of energy, the mass of adipose tissue increases through a process of increasing the number and volume of the adipocytes.

The composition for preventing and treating the lipid-related metabolic diseases may include a pharmaceutically acceptable carrier. The pharmaceutically acceptable carrier may include physiological saline, polyethylene glycol, ethanol, vegetable oil, isopropyl myristate, and the like, but is not limited thereto.

In addition, the composition for preventing and treating the lipid-related metabolic diseases may be prepared as an aqueous solution for parenteral administration, and may use preferably a buffer solution such as a Hank's solution, a Ringer's solution, or physically buffered saline, and the like. An aqueous injection suspension may be added with a substrate capable of increasing the viscosity of the suspension, such as sodium carboxymethylcellulose, sorbitol or destrant.

The present disclosure provides a health food composition for preventing and improving lipid-related metabolic diseases containing, as an active ingredient, at least one selected from the group consisting of a strain of Lactobacillus plantarum ATG-K2, a culture product of the strain, and a concentrate and a dry matter of the culture product.

Further, the present disclosure provides a health food composition for preventing and improving lipid-related metabolic diseases containing, as an active ingredient, at least one selected from the group consisting of a strain of Lactobacillus plantarum ATG-K6, a culture product of the strain, and a concentrate and a dry matter of the culture product.

The health food composition for preventing and improving the lipid-related metabolic diseases includes all types of functional foods, nutrient supplements, health foods, and food additives. The health food composition may be prepared in various forms according to a general method known in the art.

For example, the health food composition may be prepared in the form of probiotics, yogurt, drink or juice, etc. including Lactobacillus plantarum ATG-K2 or ATG-K6 of the present disclosure to be drunken or granulated, encapsulated, and powdered to be taken. In addition, the Lactobacillus plantarum ATG-K2 or ATG-K6 of the present disclosure may be mixed with a known active ingredient to be prepared in the form of the composition.

The composition may reduce a body weight gain rate and body fat, which are obesity indexes.

As can be seen in Table 3 and FIG. 2, in Examples below, it was confirmed that the body weight and the fat globule size of epididymal fat increased by a high fat/high fructose diet were reduced by administering Lactobacillus plantarum ATG-K2 and ATG-K6. Accordingly, it can be seen that Lactobacillus plantarum ATG-K2 and ATG-K6 may serve as anti-obesity materials that reduce the body weight gain rate and the body fat.

The composition may reduce the enzyme activities of ALT, AST and ALP, which are liver damage indexes.

ALT is alanine aminotransferase, AST is aspartate aminotransferase, and ALP is alkaline phosphatase, and high levels thereof mean liver damage, and low levels thereof mean restoration of a liver function.

As can be seen in FIG. 3, through the following Examples, it was confirmed that ALT, AST and ALP increased by the high fat/high fructose diet were statistically significantly improved by administering Lactobacillus plantarum ATG-K2 and ATG-K6.

The composition may decrease serum triglyceride (serum TG) and serum total cholesterol (serum TC), and increase serum HDL cholesterol.

There are four main types of lipids in the blood: total cholesterol, low-density lipoprotein (LDL) cholesterol, high-density lipoprotein (HDL) cholesterol, and triglyceride. The low-density lipoprotein (LDL) cholesterol is accumulated on the wall of blood vessel to cause arteriosclerosis, which causes cardiovascular and cerebrovascular diseases. The high-density lipoprotein (HDL) cholesterol serves to transport cholesterol accumulated on the wall of blood vessel to the liver to prevent arteriosclerosis. The total cholesterol is a generic term for low-density lipoprotein cholesterol and high-density lipoprotein cholesterol, and the triglyceride is produced to store excess energy consumed with food and normally stored in adipocytes and then released if necessary and used as an energy source.

In addition, after leptin is secreted from adipose tissue, the leptin acts in the brain to suppress appetite and activate metabolism in the body, thereby reducing body weight. When the adipocytes increase, the concentration of leptin increases to suppress hunger and stop food intake. Adiponectin is a type of protein secreted from adipocytes and serves as a decisive factor to improve insulin resistance. Therefore, the adiponectin is known as a material capable of treating obesity and diabetes. It was also found to have a function of preventing arteriosclerosis. In the case of the obesity, the amount of adiponectin in the blood is decreased, and a decrease in body fat increases the production of adiponectin.

Superoxide dismutase (SOD) is one of enzyme-based materials involved in an antioxidant mechanism, and is an enzyme having a strong antioxidant action. Glutathione peroxidase (GPx) is an enzyme that functions as an antioxidant to protect cells from being damaged by decomposing hydrogen peroxide produced in the body. Catalse (CAT) is also an enzyme having an antioxidant function.

Therefore, the reducing of serum triglyceride, serum total cholesterol, fasting serum glucose and leptin, and the increasing of blood HDL cholesterol, adiponectin, superoxide dismutase (SOD), glutathione peroxidase (GPx), and catalse (CAT) mean that it is effective in the prevention and treatment of lipid-related metabolic diseases.

In the following Examples, blood lipid levels were measured to determine the relation in fat metabolism of Lactobacillus plantarum ATG-K2 and ATG-K6. As can be seen in FIG. 4, by the high fat/high fructose diet, serum triglyceride, serum total cholesterol, fasting serum glucose and leptin were increased, and blood HDL cholesterol, adiponectin, superoxide dismutase (SOD), glutathione peroxidase (GPx), and catalse (CAT) were decreased. Thereafter, it was confirmed that by administering Lactobacillus plantarum ATG-K2 or ATG-K6, serum triglyceride, serum total cholesterol, fasting serum glucose and leptin were decreased, and blood HDL cholesterol, adiponectin, superoxide dismutase (SOD), glutathione peroxidase (GPx), and catalse (CAT) were significantly increased. In particular, it can be seen that HDL cholesterol is increased by administering Lactobacillus plantarum ATG-K2 or ATG-K6, compared to a silymarin-administered group as a positive control to have a good effect.

The composition may decrease hepatic triglyceride and total cholesterol, and decrease hepatic lipid peroxidation (MDA) .

The lipid peroxidation is increased due to an increase in intracellular oxidative stress, that is, an increase in free radical production and a decrease in antioxidant defense, but this reaction is recognized as one of the most important mechanisms of liver damage caused by various toxic compounds or drugs. The lipid peroxidation occurs when unsaturated fatty acids, a lipid component, are exposed to oxygen, and in particular, in-vivo peroxidation occurs easily in cell membranes rich in unsaturated fatty acids and phospholipids, such as mitochondria, microsomes, red blood cells and platelets.

Therefore, the reduction of hepatic triglyceride and hepatic total cholesterol and the reduction of hepatic lipid peroxidation (MDA) mean that it is effective in the prevention and treatment of lipid-related metabolic diseases.

As a result of measuring triglyceride and total cholesterol, which are lipid metabolism-related markers in liver tissue through the following Examples, as illustrated in FIG. 5, it was confirmed that hepatic triglyceride and hepatic total cholesterol were increased by the high fat/high fructose diet, and the hepatic triglyceride and the hepatic total cholesterol were significantly decreased by administering Lactobacillus plantarum ATG-K2 or ATG-K6.

In addition, as a result of measuring hepatic lipid peroxidation (MDA), it can be seen that the lipid peroxidation content increased by the high fat/high fructose diet was significantly reduced by administration of Lactobacillus plantarum ATG-K2 or ATG-K6.

The composition may reduce the expression of sterol regulatory element binding transcription factor 1 (SREBP-1c) mRNA, CCAAT/enhancer-binding protein alpha (C/EBP-α) mRNA and fatty acid synthase (FAS) mRNA, which are lipogenesis-related indexes.

The SREBP-1c, the C/EBP-α and the FAS are lipogenesis-related indexes, and the reduction in mRNA expression of SREBP-1c, C/EBP-α and FAS is effective in the prevention and treatment of lipid-related metabolic diseases.

As a result of the analysis through the following Examples, as shown in FIG. 6, it was confirmed that the mRNA expression of SREBP-1c, C/EBP-α and FAS was decreased in a group administered with Lactobacillus plantarum ATG-K2 or ATG-K6 as compared with a high fat/high fructose diet induced group.

The composition may decrease the expression of acetyl coA carboxylase (ACC) mRNA, which is a fatty acid oxidation-related index, and increase the expression of Carnitine palmitoyltransferase 1 (CPT-1) mRNA.

ACC and CPT-1 are fatty acid oxidation-related indexes, and means that a decrease in expression of ACC mRNA and an increase in expression of CPT-1 mRNA are effective in preventing and treating lipid-related metabolic diseases.

As a result of analysis through the following Examples, as illustrated in FIG. 7, it was confirmed that the mRNA expression of CPT-1 decreased and the ACC mRNA expression increased.

The composition may increase a Bacteroidetes group and inhibit a Firmicutes group in an intestinal flora.

The human intestinal microbiota is divided into four classes: gram-negative bacteria, Bacteroidetes and Proteobacteria, and gram-positive bacteria, Firmicutes and Actinobacteria. Bacteroidetes consisting of the species Bacteroides, and Firmicutes, mostly belonging to the class Clostridia, account for the majority of the classes, with less than 10% of the classes is the rest. In previous studies, when the high-fat/high-protein diet was provided to wild-type mice, the human intestinal microbiota was changed to a microbiota with increased Firmicutes and decreased Bacteroidetes. Even with the same caloric intake, sterile rats receiving intestinal microorganisms from obese rats gained more body weight than rats receiving intestinal microorganisms from lean rats. The result means that the intestinal microorganisms play an important role in energy storage and obesity occurrence. An increase in a ratio of Firmicutes/Bacteroidetes is also observed even in the intestinal microflora of obese people, but when a lowcarbohydrate or low-fat diet is supplied, the fraction of Bacteroidetes increases.

Accordingly, it can be seen that the increase in the Bacteroidetes group and the inhibition of the Firmicutes group among the intestinal flora are effective in preventing and treating the lipid-related metabolic diseases.

As a result of comparative analysis of the intestinal flora of a high fat/high fructose diet-induced group and a Lactobacillus plantarum ATG-K2 or ATG-K6 administered group through the following Examples, it was confirmed that in the Lactobacillus plantarum ATG-K2 or ATG-K6 administered group, the Bacteroidetes group was increased, and the Firmicutes group was inhibited to be changed to the intestinal flora compared to the high fat/high fructose diet-induced group.

When describing these results collectively, it can be seen that the Lactobacillus plantarum ATG-K2 or ATG-K6 1) decreases the enzyme activities of ALT, AST and ALP, which are liver damage indexes, 2) decreases serum triglycerides, serum total cholesterol, fasting serum glucose, and leptin and increases blood HDL cholesterol, adiponectin, SOD, GPx and CAT, and 3) decreases hepatic triglyceride and hepatic total cholesterol and increases hepatic lipid peroxidation (MDA). In addition, it can be seen that the Lactobacillus plantarum ATG-K2 or ATG-K6 4) decreases the expression of SREBP1c mRNA, C/EBP-α mRNA and FAS mRNA, 5) decreases the expression of ACC mRNA and increases the expression of CPT-1 mRNA, and 6) increases the Bacteroidetes group and inhibits the Firmicutes group in the intestinal flora, so that it is effective in the prevention and treatment of lipid-related metabolic diseases.

### Examples

Hereinafter, the present disclosure will be described in detail by Examples and Experimental Examples. However, the following Examples and Experimental Examples are just illustrative of the present disclosure, and the contents of the present disclosure are not limited to the following Examples and Experimental Examples.

Hereinafter, in Examples and Experimental Examples of the present disclosure, Lactobacillus plantarum ATG-K2 or ATG-K6 may mean containing a strain of Lactobacillus plantarum ATG-K2 or ATG-K6, a culture product of the strain, a concentrate of the culture product, a dry matter of the culture product, or a mixture thereof as an active ingredient.

### Experimental materials

### 1. Preparation of Lactobacillus plantarum ATG-K2 and ATG-K6

Lactobacillus plantarum ATG-K2 (Accession No. KCTC 13577BP) and Lactobacillus plantarum ATG-K6 (Accession No. KCTC 13570BP) as fermented vegetable-derived lactic acid bacteria were cultured in a Man Rogosa Sharpe (MRS) medium (Difco Laboratories, USA) at 37°C for 16 hours. K2 and K6 cells were collected by centrifugation at 8,000 rpm at 4°C for 15 minutes and washed three times with phosphate buffered saline (PBS). Finally, the cells were suspended in 12.5% trehalose, 10% skim milk, 0.125% carboxymethyl cellulose (w/v) solution, and freeze-dried using an FD8508 freeze dryer (IlShinBioBase, Korea). The dried powder was dissolved in PBS at a concentration of 5 × 10⁸ CFU for each strain and used.

### 2. Experimental design

Experimental animals were classified into groups by a randomized complete block design after receiving 6-week-old Wistar rats from ORIENT Bio (Gyeonggi-do, Korea), and confirming that all subjects had no adverse reactions over an adaptation period of 1 week. A breeding environment was maintained at a temperature of 20 to 25°C and humidity of 50 to 55% for 12 hours, and solid feed and water were freely ingested.

Experimental groups were divided into a normal diet control group (NC, 10% calories from fat; D12450B; Research Diets Inc) of 9 rats, and a high fat/high fructose diet control group (HC) fed with 45 % high fat diet (45 % calories from fat; D12451; Research Diets Inc) and 10% high fructose drinking water to induce non-alcoholic fatty liver for 8 weeks. After an induction period, a fatty liver induced group was re-divided into a total of 5 groups of a normal diet control group (Normal diet Control), a high fat/high fructose control group (HF/HF diet control, HC), a positive control group (Silymarin 100 mg/kg/b.w., positive control, PC), a Lactobacillus plantarum ATG-K2 group (K2), and a Lactobacillus plantarum ATG-K6 group (K6) by a randomized complete block design, and each test material was orally administered at 10:30 a.m. every day for 8 weeks. The body weight was measured once a week, and the diet was self-fed, and weights of an amount to be provided every feeding and the remaining amount were measured to confirm the diet efficiency between groups.

Silymarin administered to the positive control group functions as a strong antioxidant to have an antioxidant effect, and protects the cell membrane of the liver to protect the liver from toxins and help the liver to regenerate.

All laboratory animal breeding and management was approved by the Animal Experimental Ethics Committee of Gachon University and was performed in compliance with the relevant regulations (approval number: GIACUC-R2019014).

### 3. Treatment of laboratory animals

After breeding, the experimental animals were sacrificed after fasting for 12 hours. Blood collected through a cardiac puncture was centrifuged at 3,000 rpm for 15 minutes at 4°C to collect serum, and then the collected serum was stored at - 80°C until analysis. Organ tissues were immediately removed after blood collection, washed with physiological saline, dried with filter paper, and weighed, and thereafter, all samples were stored in a deep freezer at 80°C and used.

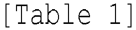

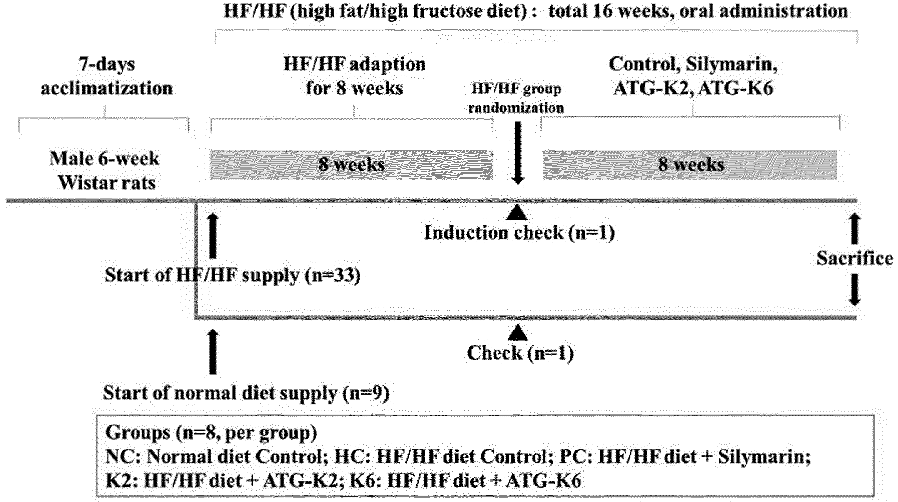

### Experimental method

The following health-related markers were measured by oral administration of Lactobacillus plantarum ATG-K2 or ATG-K6 to rats induced with non-alcoholic fatty liver by a high fat/high fructose diet.
- Measurement of body weight and weights of organs (liver and epididymal fat)
- Hepatic pathological test (H&E staining, Oil-Red O staining)
- Measurement of enzymes related to liver functions (ALT, AST, and ALP)
- Measurement of markers related to blood lipid levels (serum triglycerides, serum total cholesterol, serum HDL cholesterol, fasting serum glucose, leptin, adiponectin, SOD, GPx, and CAT)
- Measurement of hepatic lipid metabolism levels (hepatic triglycerides, hepatic total cholesterol, and MDA)
- Measurement of lipogenesis-related mRNAs (SREBP-1c, FAS, and C/EBP)
- Measurement of fatty acid oxidation-related mRNAs (ACC, and CPT-1)
- Measurement of changes in intestinal flora

### (1) Measurement of anti-obesity effect

To measure an anti-obesity effect, the body weight gain rate and the fat globule size of epididymal fat, which were obesity indexes, were confirmed.

### (2) Measurement of liver damage indexes

In order to confirm the improvement of the liver functions of fermented vegetable-derived lactic acid bacteria, aspartate aminotransferase (AST), alanine aminotransferase (ALT), and alkaline phosphate (ALP), which were liver damage indexes, in serum of experimental animals were measured by using an assay kit (Asanpharm, Hwaseong, Korea), respectively.

### (3) Measurement of serum indexes

Triglyceride (TG), total cholesterol (TC), and high density lipoprotein (HDL) cholesterol were measured using TG-S, T-CHO, and HDL-CHO kits (Asanpharm, Hwaseong, Korea), respectively.

The fasting blood glucose was measured using a blood glucose meter kit (Handok, Seoul, Korea), and the adiponectin and the leptin were measured using an enzymelinked immunosorbent assay (ELISA) kit (R&D system, Minneapolis, MN, USA).

The antioxidant indexes SOD, GPx, and CAT were measured using a superoxide dismutase activity colorimetric assay kit (BlueGene Biotech, Shanhai, China), a glutathione peroxidase assay kit (BlueGene Biotech, Shanhai, China), and a catalase assay kit (BlueGene Biotech, Shanhai, China), respectively.

### (4) Measurement of liver tissue indexes

Hepatic TG and TC were measured to measure the lipid content in liver tissue of experimental animals. Hepatic lipids were extracted using a Folch method and then analyzed using TG-S and T-CHO kits (Asanpharm, Hwaseong, Korea). MDA in liver tissue was pre-treated using a lipid hydroperoxide (LPO) assay kit, added with 500 µl of a sample, 450 µl of a chloroform-methanol solvent mixture, and 50 µl of Chromogen (FTS Reagent 1, 2) in a 96-well plate, respectively, and then cultured at room temperature for 5 minutes, and the absorbance was measured at 500 nm using an ELISA (Biolog, USA).

### (5) Hepatic pathological test (H&E staining, Oil-Red O staining)

After a hepatic sample was immobilized using 10% formalin (Sigma-Aldrich, Co., USA), paraffin tissue pieces were prepared with a thickness of 3 to 4 µm through an ethyl alcohol dehydration process and stained with hematoxylin and eosin (H&E), and then histological analysis was performed. For histopathological pictures, representative pictures for each lesion site were selected, and low magnification (x100) and high magnification (x400) pictures were taken for each slide.

Gun-sucrose-fixed liver sections were stained with Oil-red O and observed through a microscope, and pictures were taken.

### (6) mRNA and expression level

For sterol regulatory element binding transcription factor 1 (SREBP-1c), CCAAT/enhancer-binding protein alpha (C/EBP-α), and fatty acid synthase (FAS) as signaling materials involved in adipogenesis in liver tissue, and acetyl coA carboxylase (ACC) and carnitine palmitoyltransferase 1 (CPT-1) involved in fatty acid oxidation, mRNA expression was analyzed using real-time reverse transcription (RT)-polymerase chain reaction (PCR). The liver tissue was homogenized with a homogenizer (Polyton PT-MR 3100, Kinematica AG, Luzern, Switzerland), total RNA was isolated with an RNA extraction kit (iNtRON Biotechnology, Gyeonggi-do, Korea), and then cDNA was synthesized with an iScript cDNA synthesis kit (BioRad, Hercules, CA, USA). Real-time RT-PCR was performed with the synthesized cDNA and analyzed using SYBR Green Master Mix (TaKaRa Bio, Otsu, Japan) with ABI QuantStudio 3 (Applied Biosystems, Foster City, USA) equipment. The primer sequences used in the experiments of the present disclosure were shown in Table 2, and the mRNA expression was performed using β-actin as a control.

**[Table 2]**

| **Gene** | **Forward (5'-3')** | **Reverse (5'-3')** |
|---|---|---|
| SREBP-1c | CCC TGC GAA GTG CTC ACA A | GCG TTT CTA CCA CTT CAG GTT TCA |
| FAS | GCT GCT ACA AAC AGG ACC ATC AC | TCT TGC TGG CCT CCA CTG \|AC |
| C/EBPα | GCC AAG AAG TCG GTG GAT AA | CCT TGA CCA AGG AGC TCT CA |
| ACC | CAA TCC TCG GCA CAT GGA GA | GCT CAG CCA ACC GGA TGT AGA |
| CPT-1 | CCA TCT CTT CTG CCT CTA TGT | GTC AGG GTT TTT CTC AAA GTC |
| β-actin | GAT TAC TGC CCT GGC TCC TA | TCA TCG TAC TCC TGC TTG CT |

| | | |
|---|---|---|
| **SREBP-1c, Sterol regulatory element binding protein 1c; FAS, Fatty acid synthase; C/EBPα, CCAAT/enhancer-binding protein alpha; ACC, Acetyl-CoA carboxylase; CPT-1, Carnitine palmitoyltransferase-1.** | | |

### (7) Statistical analysis

All values were expressed as mean ± standard deviation, and for statistical analysis, one-way ANOVA was performed, and then a post-hoc test was performed using a Duncan's multiple range test to measure significance between groups. All statistical processes were evaluated using SPSS 25 (SPSS Inc., Chicago, Illinois, USA), and when a p-value was 0.05 or less, it was determined to be statistically significant.

### Analysis of experimental results

### 1. Body weight gain rate

Referring to Table 3, it can be seen that the high fat/high fructose diet induced body weight gain in rats, and a Lactobacillus plantarum ATG-K2 or ATG-K6 diet group had a low body weight gain rate.

**[Table 3] Body weight gain rate of each group**

| **Groups** | **Initial Body Weight** | **Body Weight after Randomization (A)** | **Terminal Body Weight (B)** | **Body Weight Gain (B-A)** |
|---|---|---|---|---|
| NC | 194.0 ± 8.4 | 485.5 ± 38.9 ^{a} | 622.8 ± 55.6 ^{a} | 137.2 ± 30.8 ^{a} |
| HC | | 522.4 ± 29.5 ^{b} | 733.3 ± 87.2 ^{b} | 210.9 ± 63.1 ^{b} |
| PC | 194.0 ± 7.6 | 522.4 ± 28.6 ^{b} | 680.4 ± 57.5 ^{ab} | 158.0 ± 35.9 ^{a} |
| K2 | | 521.9 ± 31.1 ^{b} | 677.9 ± 64.8 ^{ab} | 156.0 ± 37.7^{a} |
| K6 | | 523.6 ± 27.1 ^{b} | 697.1 ± 55.6 ^{b} | 173.5 ± 30.9 ^{a} |

| | | | | |
|---|---|---|---|---|
| **NC, Normal diet control; HC, high fat/high fructose (HF/HF) diet control; PC, HF/HF diet with silymarin; K2, HF/HF diet with ATG-K2; K6, HF/HF diet with ATG-K6.** | | | | |

### 2. Liver health improvement effect

In order to examine a liver health improvement effect of Lactobacillus plantarum ATG-K2 or ATG-K6 in a fatty liver-induced rat animal model, liver tissue was analyzed by H&E staining. As can be seen from FIG. 1, in the high fat/high fructose diet group, compared to the normal diet group, fat was accumulated to induce non-alcoholic fatty liver, and it was confirmed with the naked eye that the non-alcoholic fatty liver-induced fatty liver was remarkably improved by Lactobacillus plantarum ATG-K2 or ATG-K6. In addition, the findings were confirmed once again through Oil-Red O staining.

### 3. Fat globule size in epididymal fat

As can be seen in FIG. 2, when comparing epididymal fat through H&E staining, it was confirmed that in the high fat/high fructose diet group, the fat globule size was increased, and the fat globule size in the epididymal fat was reduced by Lactobacillus plantarum ATG-K2 or ATG-K6. Therefore, it can be seen that Lactobacillus plantarum ATG-K2 or ATG-K6 is effective in reducing body fat.

### 4. Enzyme activities of ALT, AST, and ALP as liver damage indexes

In order to determine effects of Lactobacillus plantarum ATG-K2 or ATG-K6 on liver function and damage, enzyme activities of serum ALT, AST, and ALP were measured. As a result, as can be seen in FIG. 3, it can be seen that ALT, AST and ALP increased by the high fat/high fructose diet were statistically significantly improved by administering Lactobacillus plantarum ATG-K2 and ATG-K6.

### 5. Measurement of blood lipid levels

In order to determine the relevance of fat metabolism of Lactobacillus plantarum ATG-K2 or ATG-K6, serum triglyceride (serum TG), serum total cholesterol (serum TC), serum HDL cholesterol, fasting serum glucose, leptin, adiponectin , superoxide dismutase (SOD), glutathione peroxidase (GPx), and catalse (CAT) were measured.

As a result, as illustrated in FIG. 4, serum triglyceride, serum total cholesterol, fasting serum glucose, and leptin were increased by the high fat/high fructose diet, and significantly reduced by Lactobacillus plantarum ATG-K2 or ATG-K6. HDL cholesterol, adiponectin, SOD, GPx, and CAT were decreased by the high fat/high fructose diet, and increased when Lactobacillus plantarum ATG-K2 or ATG-K6 was administered. In particular, it can be seen that HDL cholesterol is increased by administering Lactobacillus plantarum ATG-K2 or ATG-K6, compared to a silymarin-administered group as a positive control to have a good effect.

### 6. Measurement of lipid metabolism in liver tissue

Hepatic triglyceride and total cholesterol as markers related to lipid metabolism were measured in liver tissue. As a result, as can be seen from FIG. 5, hepatic triglyceride and total cholesterol were increased by the high fat/high fructose diet, and were significantly decreased by administering lactic acid bacteria derived from fermented vegetables. In addition, as a result of measuring hepatic lipid peroxidation (MDA), it can be seen that the lipid peroxidation content increased by the high fat/high fructose diet was significantly reduced by administering lactic acid bacteria derived from fermented vegetables.

### 7. Analysis of mRNA expression of indexes related to lipid metabolism

(1) In order to examine a molecular mechanism of lactic acid bacteria derived from fermented vegetables, the expression of lipogenesis-related genes SREBP-1c, FAS, and C/EBP was examined by RT-PCR and immunoblotting. As can be seen in FIG. 6, as a result of the analysis, the mRNA expression of lipid metabolism-related indexes SREBP-1c, FAS, and C/EBP was reduced in a Lactobacillus plantarum ATG-K2 or ATG-K6 administered group as compared with a non-alcoholic fatty liver induced group.
(2) In order to determine an effect of Lactobacillus plantarum ATG-K2 or ATG-K6 on hepatic lipid metabolism, the expression of fatty acid oxidation-related genes ACC and CPT-1 was measured by qRT-PCT. As illustrated in FIGS. 7A and 7B, the expression of ACC mRNA was lower in the Lactobacillus plantarum ATG-K2 or ATG-K6 administered group than the non-alcoholic fatty liver induced group, and the CPT-1 mRNA level was higher in the Lactobacillus plantarum ATG-K2 or ATG-K6 administered group than the non-alcoholic fatty liver induced group.

In order to determine whether Lactobacillus plantarum ATG-K2 or ATG-K6 activates AMPK signaling, immunoblotting was performed. As confirmed in FIGS. 7C and 7D, phosphorylation levels were improved in both markers after treatment with Lactobacillus plantarum ATG-K2 or ATG-K6. In addition, as shown in FIG. 7E, the expression of CPT-1 protein was also higher in the Lactobacillus plantarum ATG-K2 or ATG-K6 treated group than the non-alcoholic fatty liver induced group.

### 8. Measurement of changes in intestinal flora

An effect of Lactobacillus plantarum ATG-K2 or ATG-K6 on the intestinal microflora was analyzed. The feces for each experimental group were freshly collected in 3 experimental tubes and immediately frozen at - 80°C. An amplicon sequence of a V3-V4 region of 16S rRNA was obtained from metagenomic DNA extracted from the frozen sample using a Miseq platform, next-generation sequencing equipment, and changes in the intestinal flora caused by Lactobacillus plantarum ATG-K2 or ATG-K6 were analyzed using the amplicon sequence.

As can be seen in FIG. 8, it was confirmed that Lactobacillus plantarum ATG-K2 or ATG-K6 lactic acid bacteria treated experimental groups K2 and K6 increased a Bacteroidetes group like an untreated experimental group (NC) or a silymarin treated positive control (PC) group and inhibited a Firmicutes group to exhibit an effect as probiotics that changed the intestinal flora.

So far, specific embodiments of the composition for preventing and treating the lipid-related metabolic diseases containing Lactobacillus plantarum ATG-K2 or ATG-K6 according to an exemplary embodiment of the present disclosure have been described, but it is apparent that various modifications are possible without departing from the scope of the present disclosure.

Therefore, the scope of the present disclosure should not be limited to the exemplary embodiments and should be defined by the appended claims and equivalents to the appended claims.

In other words, the exemplary embodiments described above are illustrative in all aspects and should be understood as not being restrictive, and the scope of the present disclosure is represented by appended claims to be described below rather than the detailed description, and it is to be interpreted that the meaning and scope of the appended claims and all changed or modified forms derived from the equivalents thereof are included within the scope of the present disclosure.

### [Industrial Applicability]

According to the present disclosure, the present disclosure relates to a composition for preventing and treating lipid-related metabolic diseases, including, as an active ingredient, Lactobacillus plantarum ATG-K2 or ATG-K6 isolated from fermented vegetables. As a result of measuring a liver health-related index and an anti-obesity index by oral administration of Lactobacillus plantarum ATG-K2 or ATG-K6, it can be easily used as a composition for preventing and treating lipid-related metabolic diseases because of its excellent effect of improving the indexes.

### [Sequence List Text]

SEQ ID NO: 1 is a forward primer nucleotide sequence for real-time reverse transcription polymerase chain reaction of SREBP-1C.
SEQ ID NO: 2 is a reverse primer nucleotide sequence for real-time reverse transcription polymerase chain reaction of SREBP-1C.
SEQ ID NO: 3 is a forward primer nucleotide sequence for real-time reverse transcription polymerase chain reaction of FAS.
SEQ ID NO: 4 is a reverse primer nucleotide sequence for real-time reverse transcription polymerase chain reaction of FAS.
SEQ ID NO: 5 is a forward primer nucleotide sequence for real-time reverse transcription polymerase chain reaction of C/EBPα.
SEQ ID NO: 6 is a reverse primer nucleotide sequence for real-time reverse transcription polymerase chain reaction of C/EBPα.
SEQ ID NO: 7 is a forward primer nucleotide sequence for real-time reverse transcription polymerase chain reaction of ACC.
SEQ ID NO: 8 is a reverse primer nucleotide sequence for real-time reverse transcription polymerase chain reaction of ACC.
SEQ ID NO: 9 is a forward primer nucleotide sequence for real-time reverse transcription polymerase chain reaction of CPT-1.
SEQ ID NO: 10 is a reverse primer nucleotide sequence for real-time reverse transcription polymerase chain reaction of CPT-1.
SEQ ID NO: 11 is a forward primer nucleotide sequence for real-time reverse transcription polymerase chain reaction of β-actin.
SEQ ID NO: 12 is a reverse primer nucleotide sequence for real-time reverse transcription polymerase chain reaction of β-actin.

## Claims

1. A composition for preventing and treating lipid-related metabolic diseases containing, as an active ingredient, at least one selected from the group consisting of a strain of Lactobacillus plantarum ATG-K2 (accession No. KCTC 13577BP), a culture product of the strain, and a concentrate and a dry matter of the culture product.

2. A composition for preventing and treating lipid-related metabolic diseases containing, as an active ingredient, at least one selected from the group consisting of a strain of Lactobacillus plantarum ATG-K6 (accession No. KCTC 13570BP), a culture product of the strain, and a concentrate and a dry matter of the culture product.

3. A health food composition for preventing and improving lipid-related metabolic diseases containing, as an active ingredient, at least one selected from the group consisting of a strain of Lactobacillus plantarum ATG-K2 (accession No. KCTC 13577BP), a culture product of the strain, and a concentrate and a dry matter of the culture product.

4. A health food composition for preventing and improving lipid-related metabolic diseases containing, as an active ingredient, at least one selected from the group consisting of a strain of Lactobacillus plantarum ATG-K6 (accession No. KCTC 13570BP), a culture product of the strain, and a concentrate and a dry matter of the culture product.

5. The composition of any one of claims 1 to 4, wherein the composition reduces a body weight gain rate and body fat, which are obesity indexes.

6. The composition of any one of claims 1 to 4, wherein the composition reduces enzyme activities of ALT, AST and ALP, which are liver damage indexes.

7. The composition of any one of claims 1 to 4, wherein the composition decreases serum triglyceride, serum total cholesterol, fasting serum glucose and leptin, and increases blood HDL cholesterol, adiponectin, superoxide dismutase (SOD), glutathione peroxidase (GPx) and catalse (CAT) .

8. The composition of any one of claims 1 to 4, wherein the composition decreases hepatic triglyceride and hepatic total cholesterol, and decreases hepatic lipid peroxidation (MDA) .

9. The composition of any one of claims 1 to 4, wherein the composition decreases the expression of sterol regulatory element binding transcription factor 1 (SREBP-1c) mRNA, CCAAT/enhancer-binding protein alpha (C/EBP-α) mRNA and fatty acid synthase (FAS) mRNA, which are lipogenesis-related indexes.

10. The composition of any one of claims 1 to 4, wherein the composition decreases the expression of acetyl coA carboxylase (ACC) mRNA, which is a fatty acid oxidation-related index, and increases the expression of Carnitine palmitoyltransferase 1 (CPT-1) mRNA.

11. The composition of any one of claims 1 to 4, wherein the composition increases a Bacteroidetes group and inhibits a Firmicutes group in an intestinal flora.
